# EUROPÄISCHE PATENTSCHRIFT

(11) **EP 0 757 667 B1**
(45) Veröffentlichungstag und Bekanntmachung des Hinweises auf die Patenterteilung: **19.04.2000**
(21) Anmeldenummer: 95917931.8
(22) Anmeldetag: 18.04.1995
(51) Int. Cl.: C07B 39/00, C07C 25/13, C07C 17/12

(54) **VERFAHREN ZUR HERSTELLUNG BROM ENTHALTENDER AROMATISCHER VERBINDUNGEN, NEUE BROM ENTHALTENDE VERBINDUNGEN UND IHRE VERWENDUNG ALS ZWISCHENPRODUKTE BEI DER SYNTHESE VON WIRKSTOFFEN**
PROCESSES FOR PREPARING AROMATIC, BROMINE-CONTAINING COMPOUNDS, NOVEL BROMINE-CONTAINING COMPOUNDS, AND THEIR USE AS INTERMEDIATE PRODUCTS IN THE SYNTHESIS OF ACTIVE AGENTS
PROCEDES DE PREPARATION DE COMPOSES AROMATIQUES CONTENANT DU BROME, NOUVEAUX COMPOSES CONTENANT DU BROME, ET LEUR UTILISATION COMME PRODUITS INTERMEDIAIRES DANS LA SYNTHESE DE PRINCIPES ACTIFS

(30) Priorität: 28.04.1994 DE 4414841
(43) Veröffentlichungstag der Anmeldung: 12.02.1997
(73) Patentinhaber: BAYER AG, 51368 Leverkusen (DE)
(72) Erfinder: HAGEMANN, Hermann, D-51375 Leverkusen (DE)
(86) Internationale Anmeldenummer: EP9501439
(87) Internationale Veröffentlichungsnummer: WO9529886

(56) Entgegenhaltungen:
- EP-A- 0 024 516
- EP-A- 0 431 373
- FR-A- 1 063 077
- FR-A- 2 105 250

## Beschreibung

Die vorliegende Erfindung betrifft Verfahren zur Herstellung Brom enthaltender aromatischer Verbindungen, neue Brom enthaltende aromatische Verbindungen und ihre Verwendung als Zwischenprodukte bei der Synthese von Wirkstoffen.

Verfahren zur Bromierung aromatischer Verbindungen sind beispielsweise in FR-A-1 063 077 und FR-A-2 105 250 beschrieben.

FR-A-1 063 077 lehrt die Bromierung aromatischer Verbindungen, die nicht mehr als drei Substituenten aufweisen, unter Verwendung eines Gemisches annähernd äquimolarer Mengen von Brom und Chlor als Bromierungsreagenz. Trotz langer Reaktionszeiten bzw. hoher Temperaturen, sind die zu erzielenden Umsätze nicht zufriedenstellend

Auch FR-A-2 105 250 beschreibt die Bromierung aromatischer Verbindungen mittels eines Gemisches aus Brom und Chlor. Es wird vor allem die Darstellung von Flammschutzmitteln ausgehend von Benzol, Toluol, Phenol und Diphenyl behandelt. Auch hier sind lange Reaktionszeiten nötig und die Selektivität ist nur mäßig.

Erfindungsgemäße Verfahren zur Herstellung bekannter und neuer Brom enthaltender Verbindungen sind durch spezielle Bedingungen gekennzeichnet.

Es ist dadurch möglich, aromatische Verbindungen sehr selektiv und mit hohen Umsätzen unter milden Reaktionsbedingungen in kurzen Zeiten zu bromieren. Es können auch aromatische Verbindungen bromiert werden, die bereits mehr als drei Substituenten tragen.

Das erfindungsgemäße Verfahren ist aber generell zur Bromierung aromatischer Systeme geeignet. Manche aromatische Systeme sind nur unter relativ drastischen Bedingungen elektrophil zu bromieren. In diesen Fällen findet das erfindungsgemäße Verfahren bevorzugt Anwendung. Hierzu gehören beispielsweise Aromaten, die bereits durch elektronegative Gruppen substituiert und daher desaktiviert sind. Als konkretes Beispiel sei Nitrobenzol genannt, aber auch neue, Brom enthaltende aromatische Verbindungen sind durch das erfindungsgemäße Verfahren zugänglich.

Als neue Brom enthaltende aromatische Verbindungen sind sowohl Verbindungen gemeint, die das Brom im aromatischen Kern enthalten als auch Verbindungen, die das Brom sowohl im aromatischen Kern als auch im Trihalogenmethylsubstituenten enthalten.

Gegenstand der vorliegenden Erfindung sind daher auch Verbindungen der allgemeinen Formel 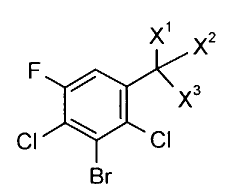 in welcher
X¹, X² und X³ für Halogen stehen,
mit der Maßgabe, daß mindestens ein Substituent aus der Gruppe X¹, X² und X³ nicht Fluor sein kann.

Bevorzugt ist 3-Brom-2,4-dichlor-5-fluorbenzotrichlorid.

Der aromatische Kern solcher Brom enthaltender aromatischer Verbindungen besitzt vorzugsweise weitere elektronenziehende Substituenten wie beispielsweise Halogene, funktionelle Gruppen auf der Oxidationsstufe einer Carbonsäure oder Nitrogruppen. Als Halogensubstituenten sind insbesondere Chlor und Fluor zu nennen und als funktionelle Gruppen auf der Oxidationsstufe einer Carbonsäure insbesondere Trihalogenmethylgruppen, vorzugsweise eine Trichlormethylgruppe. Konkrete Beispiele der erfindungsgemäßen neuen Brom enthaltenden aromatischen Verbindungen sind 3-Brom-2,4-dichlor-5-fluorbenzotrichlorid (4) und 3-Brom-2,4-dichlor-5-fluorbenzodichloridmonobromid (5), die vorteilhaft als Zwischenprodukte bei der Synthese von hochwirksamen Chinoloncarbonsäuren Verwendung finden können.

Die Verwendung als Zwischenprodukte für Chinoloncarbonsäuren erfolgt durch Hydrolyse der Trihalogenmethylgruppe zur 3-Brom-2,4-dichlor-5-fluorbenzoesäure bzw. partielle Hydrolyse zum entsprechenden 3-Brom-2,4-dichlor-5-fluorbenzoesäurechlorid bzw. -bromid.

3-Brom-2,4-dichlor-5-fluorbenzoesäure und das 3-Brom-2,4-dichlor-5-fluorbenzoylchlorid sind als Zwischenprodukte für hochwirksame Chinoloncarbonsäuren bekannt und können nach DE-A 3 631 906 dadurch hergestellt werden, daß man 2,4-Dichlor-5-fluorbenzoesäure in der 3-Position nitriert, zur Aminoverbindung hydriert und Brom über eine Sandmeyer-Reaktion einführt. Die zahlreichen und aufwendigen Reaktionsschritte machen diesen Weg sehr teuer.

Nach EP-A-0 431 373 ist es möglich, 5-Fluorbenzoesäuren durch Trichlormethylierung von Fluoraromaten und anschließende Hydrolyse der Trichlormethylsubstituenten zu erhalten. Dabei werden aber im ersten Reaktionsschritt, d.h. bei der Einführung der Trichlormethylsubstituenten maximal 70% der Theorie des gewünschten Produkts erhalten.

Es wurde nun überraschenderweise gefunden, daß man 2,4-Dichlor-5-fluorbenzotrichlorid (1), technisches Zwischenprodukt für Chinoloncarbonsäuren, unter speziellen Bedingungen mit hervorragender Raumzeitausbeute direkt in der 3-Position bromieren kann und dabei je nach Reaktionsbedingungen weitgehend einheitlich das neue 3-Brom-2,4-dichlor-5-fluorbenzo-trichlorid (4) oder die durch Brom/Chlor-Austausch in der Seitenkette gebildete analoge Dichlormonobrommethyl-Verbindung als zusätzliches neues Zwischenprodukt erhält. Beide Verbindungen lassen sich analog zu bekannten Verfahren zur Carbonsäure bzw. zum Carbonsäurehalogenid hydrolysieren.

Bei der Hydrolyse aus dem eventuell nicht umgesetzten 2,4-Dichlor-5-fluorbenzotrichlorid (1) entstehendes 2,4-Dichlor-5-fluorbenzylchlorid kann durch Destillation leicht abgetrennt werden und ist ebenfalls ein Zwischenprodukt für die Herstellung von Chinoloncarbonsäuren.

Das erfindungsgemäße Verfahren wird in Gegenwart von Aluminiumchlorid-Katalysatoren, Brom und Chlor durchgeführt. Zusätzlich muß die Möglichkeit bestehen, daß sich während der Reaktion schnell ClBr bilden kann. Als Bromierungsmittel kann hier erfindungsgemäß elementares Brom eingesetzt werden. In bevorzugter Weise wird hierfür in Gegenwart des Aluminiumchlorid-Katalysators, der zu bromierenden Verbindung und elementarem Brom Chlor in das Reaktionsgemisch eingeleitet, vorzugsweise bei einer Temperatur von 20 bis 90°C.

Als eigentliches Kernbromierungsreagenz wird in diesem Fall die Kombination der Verbindung ClBr (3) und AlCl₃ auftreten, der Komplex AlCl₄-Br^{⊕} , der sich offenbar wesentlich schneller bildet bzw. in höherer Konzentration auftritt als der entsprechende Komplex AlCl₃Br-Br^{⊕} und so die überragende erfindungsgemäße Wirkung aufweist.

Überraschenderweise findet unter diesen Reaktionsbedingungen bei geeigneter Temperaturführung und Stöchiometrie auch nahezu keine Chlorierung des aromatischen Kerns statt.

Ohne Einleitung von Chlor erfolgt die Bildung von ClBr (3) offenbar durch Cl/Br-Austausch in der Seitenkette, wie die erfindungsgemäße Bildung von 2,4-Dichlor-5-fluorbenzodichloridmonobromid (2) und 3-Brom-2,4-dichlor-5-fluor-benzodichloridmonobromid (5) neben dem entsprechenden 3-Brom-2,4-dichlor-5-fluorbenzotrichlorid (4) unter diesen Bedingungen zeigt.

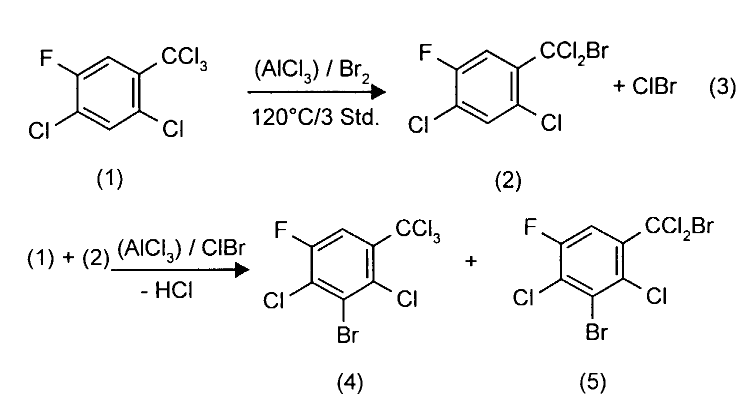

Bei einer bevorzugten Durchführungsform des Verfahrens wird die zu bromierende Verbindung zusammen mit dem Aluminiumchlorid-Katalysator vorgelegt, auf Reaktionstemperatur erhitzt und aus einem zweiten Reaktionsgefäß vorzugsweise vorerwärmtes Brom, vorzugsweise auf ca. 50°C vorerwärmt, mittels eines Chlorstroms kontinuierlich in das Reaktionsgefäß mit der zu bromierenden Verbindung eingeleitet. Da beim Einleiten von Chlor in elementares Brom offenbar sofort im Gleichgewicht mit den beiden Ausgangselementen die Verbindung ClBr (3) mit einem Siedepunkt von ca. 5°C entsteht, wird im Bromierungsgefäß offenbar ClBr in so großem Überschuß gegenüber Chlor angeboten, daß die oben beschriebene überraschend schnelle und selektive Bromierung erfolgt.

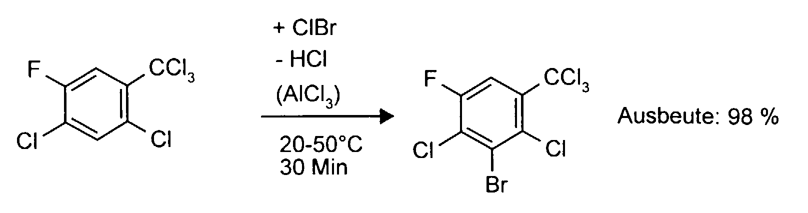

Das erfindungsgemäße Verfahren kann auch in unter den Reaktionsbedingungen inerten Lösungsmitteln durchgeführt werden, wie sie für Bromierungen aus der Literatur bekannt sind. In bevorzugter Weise wird jedoch ohne Lösungsmittel gearbeitet.

Brom wird in einer Menge von 25 bis 200 Mol-%, bezogen auf den Einsatzstoff, bevorzugt 70 bis 150 Mol-%, besonders bevorzugt 90 bis 120 Mol-% eingesetzt. Chlor oder eine Chlor abgegebende Verbindung wird in einer Menge eingesetzt, die im allgemeinen ca. 10 bis 20 Mol-% kleiner ist als die eingesetzte Brommenge, also etwa 15 bis 190 Mol-% bezogen auf die zu bromierende Verbindung, bevorzugt 60 bis 130 Mol-% und besonders bevorzugt 80 bis 100 Mol-%.

Die Katalysatormenge beträgt 0,05 bis 10 Mol-%, bezogen auf den Einsatzstoff, bevorzugt 1 bis 8 Mol-%, besonders bevorzugt 2 bis 4 Mol-%. Die Temperatur ist in Abhängigkeit von den Substituenten am aromatischen Kern in breiten Grenzen variierbar. Für die obengenannten konkreten Beispiele beträgt sie 0 bis 180°C.

Bevorzugt wird bei 10 bis 150°C, besonders bevorzugt bei 20 bis 90°C bromiert.

Die Bromierung wird im allgemeinen bei Normaldruck durchgeführt. Es ist jedoch möglich, unter Druck, beispielsweise bei bis zu 5 bar, zu arbeiten und kontinuierlich oder absatzsweise im Idealfall den entstandenen Chlorwasserstoff, in der Praxis Chlorwasserstoff im Gemisch mit Bromwasserstoff, zu entspannen.

Die Reaktionszeit für die Bromierung richtet sich nach Art und Menge des Katalysators sowie nach der Temperatur.

Zusätzlich ist die Reaktionszeit in der Praxis auch abhängig von der Ansatzgröße. Unter den bevorzugten Reaktionsbedingungen liegt sie für einen 70 mMol-Ansatz bei ca. 30 Min.

Wird die Isolierung des 3-Brom-2,4-dichlor-5-fluorbenzotrichlorids in reiner Form gewünscht, kann evtl. noch überschüssiges Brom und der Katalysator nach Beendigung der Bromierung mit wäßriger Natriumthiosulfatlösung abgetrennt und die organische Phase fraktioniert destilliert werden. Es ist nach Beendigung der Bromierung aber auch eine direkte fraktionierte Destillation möglich.

In den Fällen, bei denen unter den Bromierungsbedingungen gegen Ende der Reaktion unerwünschte Nebenprodukte auftreten, wird in bevorzugter Weise vor dem vollständigen Umsatz abgebrochen.

Die Verwendung des obengenannten konkreten Beispiels 3-Brom-2,4-dichlor-5-fluorbenzotrichlorid als Zwischenprodukt für Chinoloncarbonsäuren durch Hydrolyse der Trichlormethylgruppe zur 3-Brom-2,4-dichlor-5-fluorbenzoesäure bzw. partielle Hydrolyse zum entsprechenden 3-Brom-2,4-dichlor-5-fluorbenzoesäurechlorid wird im allgemeinen so durchgeführt, daß man das Benzotrichlorid (4) mit der entsprechenden Menge Wasser oder einem Wasser abspaltenden Mittel, wie beispielsweise Ameisensäure, zur Reaktion bringt.

Das zur Hydrolyse einzusetzende Wasser kann auch das Verdünnungswasser eines anderen Stoffes sein, beispielsweise in Form von nicht konzentrierter Schwefelsäure. Wasser oder eine wasserabgebende Substanz wird im Falle der Hydrolyse zum Säurechlorid im wesentlichen in äquimolarer Menge, bezogen auf den Ausgangsstoff, eingesetzt, beispielsweise in einer Menge von 0,8 bis 1,2 Mol, bevorzugt 0,9 bis 1,1 Mol, besonders bevorzugt 0,95 bis 1,05 Mol H₂O pro Mol Ausgangsstoff.

Die Hydrolyse wird in Gegenwart oder Abwesenheit eines gegen die Hydrolyse inerten Lösungsmittels durchgeführt. Als geeignete Lösungsmittel haben sich beispielsweise Chlorbenzol oder eines der isomeren Dichlorbenzole erwiesen. In bevorzugter Weise wird ohne Lösungsmittels gearbeitet. Die Hydrolyse kann rein thermisch oder in Gegenwart eines Katalysators durchgeführt werden. Solche Katalysatoren gehören der Gruppe der Lewis- und Brönsted-Säuren an.

Beispiele hierfür sind: FeCl₃, ZnCl₂, ZrOCl₂, H₂SO₄, SbCl₃, AlCl₃. Die Hydrolyse von Benzotrichloriden zu Benzoylchloriden ist im Prinzip bekannt (Ullmanns Encyclopädie der technischen Chemie, 4. Auflage, Band 8 (1974), S. 373). Der Zusatz eines der genannten Katalysatoren bezieht sich vor allem auf die Hydrolyse eines zwischenisolierten 3-Brom-2,4-dichlor-5-fluorbenzotrichlorids (4). Die Hydrolyse wird bei einer Temperatur von 20 bis 200°C, bevorzugt 80 bis 160°C durchgeführt.

Das isolierte Zwischenprodukt oder das gesamte Reaktionsgemisch der Bromierung wird hierzu vorgelegt, auf die Reaktionstemperatur gebracht, woraufhin die genannte Wassermenge eindosiert wird. Die Dosiergeschwindigkeit des Wassers oder der wasserabspaltenden Substanz richtet sich nach der Temperatur, der Wärmeabfuhr (exotherme Reaktion) und der Gasabfuhr.

Die Aufarbeitung erfolgt durch fraktionierte Destillation.

### Beispiel 1

Es werden 20 g (70,7 mMol) 2,4-Dichlor-5-fluorbenzotrichlorid und 400 mg Aluminiumchlorid in einem Dreihalskolben mit Rückflußkühler, Rührer, Gasein- und ausleitungsvorrichtung vorgelegt und mit 5 g (70,4 mMol) Chlor aus einer Chlorbombe 4,2 ml (81 mMol) auf ca. 50°C erwärmtes Brom aus einem zweiten Gefäß innerhalb von 30 Min. in den Dreihalskolben transsportiert. Die bei ca. 20°C vorgelegte Mischung von 2,4-Dichlor-5-fluorbenzotrichlorid und Aluminiumchlorid erwärmt sich dabei zu Beginn durch die exotherme Reaktion auf ca. 50°C und wird danach durch Kühlung bis zur Beendigung der Zudosierung von Cl₂ und Br₂, bzw. ClBr, bei ca. 30°C gehalten.

Man läßt ca. 30 Min. nachrühren, versetzt die Reaktionslösung mit 150 ml Dichlormethan und schüttelt das überschüssige Brom mit 10 %iger wäßriger Natriumthiosulfatlösung aus. Die organische Phase wird abgetrennt, mit Natriumsulfat getrocknet, filtriert und bis zu einem Druck von 20 mbar und 50°C eingeengt. Man erhält 26,2 g 3-Brom-2,4-dichlor-5-fluorbenzotrichlorid mit einer Reinheit von GC von 95,5 %. Bei einer Reinheit des eingesetzten 2,4-Dichlor-5-fluorbenzotrichlorids von 97,5 % entspricht das einer Ausbeute von ca. 98 % (Sdp. 115°/0,1 mbar).

### Beispiel 2

Es wird wie im Beispiel 1 verfahren, jedoch anstelle der wäßrigen Aufarbeitung nach Beendigung der Bromierungsreaktion auf 140°C erhitzt und mit Stickstoff überschüssiges Brom ausgetrieben. Nach Zugabe von 0,32 g (3 Mol-%) FeCl₃ wird unter guter Durchmischung bei der obengenannten Temperatur innerhalb von ca. 60 Minuten 1,26 g (70 mmol) Wasserdampf unter die Oberfläche des zu hydrolisierenden Produkts dosiert. Man läßt 30 Minuten nachreagieren und erhält 21,5 g eines Produkts, das nach GC 86,5 % 3-Brom-2,4-dichlor-5-fluorbenzoylchlorid und 6,5 % Edukt enthält. Bei einer Reinheit des eingesetzten Benzotrichlorid von 95,5 % entspricht das einer Ausbeute von 90,7 % der Theorie.

### Beispiel 3

Es wird wie in Beispiel 1 verfahren, jedoch die Reaktion mit 2 mol 2,4-Dichlor-5-fluorbenzotrichlorid durchgeführt.

Man erhält 746,4 g 3-Brom-2,4-dichlor-5-fluorbenzotrichlorid mit einer Reinheit nach GC von 95 %. Bei einer Reinheit des eingesetzten 2,4-Dichlor-5-fluorbenzotrichlorid von 97,5 % entspricht das einer Ausbeute von ca. 97,5 %.

### Beispiel 4

Es wird wie in Beispiel 2 verfahren, jedoch die Reaktion mit 2 Mol 3-Brom-2,4-dichlor-5-fluorbenzotrichlorid und mit (10 mol-%) FeCl₃ durchgeführt. Das Rohproduktgemisch wird im Vakuum destilliert und man erhält 502 g 3-Brom-2,4-dichlor-5-fluorbenzotrichlorid mit einer Reinheit nach GC von 96,7 % (Kp_{0,05} 82°-85°C). Bei einer Reinheit des eingesetzten Benzotrichlorid von 95 % entspricht das einer Ausbeute von 83 % der Theorie.

### Beispiel 5 (zum Vergleich)

Es werden 62 g 2,4-Dichlor-5-fluorbenzotrichlorid und 2,4 g AlCl₃ (8 Mol-%) in einem Dreihalskolben mit Rückflußkühler, Rührer, Gasein- und Gasausleitungsvorrichtung vorgelegt und bei ca. 20°C 40,5 g (0,25 Mol) Brom zugegeben. Man erwärmt auf ca. 110°C. Ab ca. 80°C entsteht eine Gasentwicklung, die nach 3 Stunden beendet ist. Die Aufarbeitung wird wie in Beispiel 1 durchgeführt. Man erhält 75,4 g mit folgender Zusammensetzung:
4,8 % 2,4-Dichlor-5-fluorbenzotrichlorid
4,7 % 2,4-Dichlor-5-fluorbenzodichloridmonobromid
37,5 % 3-Brom-2,4-dichlor-5-fluorbenzotrichlorid
40,2 % 3-Brom-2,4-dichlor-5-fluorbenzodichloridmonobromid
12,8 % andere Produkte.

Bei einer Reinheit des eingesetzten Benzotrichlorid von 95 % entspricht das einer Selektivität von 82,8 % bezogen auf das Zielmolekül 3-Brom-2,4-dichlor-5-fluorbenzoesäure in Beispiel 6.

### Beispiel 6

Es werden 50 g eines Gemisches von
3,5 % 2,4-Dichlor-5-fluorbenzodichloridmonobromid
2,2 % 2,4-Dichlor-5-fluorbenzotrichlorid
40,2 % 3-Brom-2,4-dichlor-5-fluorbenzodichloridmonobromid
37,7 % 3-Brom-2,4-dichlor-5-fluorbenzotrichlorid
und 2,2 g FeCl₃ (10 Mol-%) in einem Dreihalskolben mit Rührer, Rückflußkühler, Tropftrichter und Gasausleitungsvorrichtung vorgelegt. Man erwärmt auf 110°C und tropft langsam 5 g (0,27 Mol) Wasser in die Reaktionslösung. Unter zügiger Gasentwicklung beginnt die Säure auszufallen. Man läßt 1 Stunde bei 110°C nachrühren, kühlt ab, versetzt mit ca. 100 ml Wasser, saugt ab, wäscht mit Wasser neutral, trocknet bei 80°C/20 mbar und erhält 36,8 g eines Produktgemisches, das ca. 80 % 3-Brom-2,4-dichlor-5-fluorbenzosäure und ca. 7 % 2,4-Dichlor-5-fluorbenzoesäure enthält. Fp. 169° bis 170°C.

### Beispiel 7

Es werden 36 g des Säuregemisches aus Beispiel 6 und 140 ml Thionylchlorid in einem Dreihalskolben mit Rührer, Kühler, Thermometer und Gasausleitungsvorrichtung vorgelegt und langsam auf RT erwärmt. Ab ca. 50°C beginnt eine zügige Gasentwicklung und es entsteht aus der Suspension eine klare Lösung. Man läßt 1 Stunde bei RT nachreagieren, kühlt ab, destilliert und erhält 30 g eines Säurechloridgemisches, das
90,4 % 3-Brom-2,4-dichlor-5-fluorbenzoylchlorid und
6,9 % 2,4-Dichlor-5-fluorbenzoylchlorid enthält. (Kp._{0,1} 85° bis 90°C)

### Beispiel 8

Es werden 600 ml 1n NaOH in einem Dreihalskolben mit Rührer, Thermometer und Tropftrichter vorgelegt und 70 g 3-Brom-2,4-dichlor-5-fluorbenzoylchlorid (Rohprodukt aus Beispiel 2) innerhalb von 15 Minuten bei RT in die In NaOH zugegeben. Bei geringer exothermer Reaktion (bis 32°C) ist die Verseifung nach 1 Stunde beendet. Man filtriert einen geringen braunen Niederschlag ab und fällt die Säure mit 10%iger HCl aus. Die Säure wird wie in Beispiel 6 isoliert (58 g) und analog Beispiel 7 in das Säurechlorid überführt. Man erhält 51 g 3-Brom-2,4-dichlor-5-fluorbenzoylchlorid mit einer Reinheit nach GC von 99 %. Bei einer Reinheit des eingesetzten Rohproduktes von 94 % entspricht das einer Ausbeute von 83 % der Theorie (Sdp. 85°C/0,1 mbar).

### Beispiel 9 (zum Vergleich)

Es werden 5 g 2,4-Dichlor-5-fluorbenzoylchlorid und 0,2 g AlCl₃ (4 Mol-%) in einem Dreihalskolben mit Rührer, Rückflußkühler, Gasein- und Gasausleitungsvorrichtung vorgelegt und bei RT 4 g Brom zugegeben. Die Reaktionsmischung wird auf ca. 150°C erwärmt und aus einer Chlorbombe 1,5 g Cl₂ eingeleitet. Man läßt ca. 30 Minuten nachrühren und arbeitet wie in Beispiel 1 die Reaktionslösung auf. Nach GC/MS wurden nachgewiesen:
82 % Edukt
7,5 % 2,3,4-Trichlor-5-fluorbenzoylchlorid
8,2 % 3-Brom-2,4-dichlor-5-fluorbenzoylchlorid.

### Beispiele 10 und 11 (zum Vergleich)

Wird analog wie in Beispiel 9 verfahren, jedoch mit Fe-Spänen bzw. AlCl₃ als Katalysator und ohne Verwendung von Chlor, findet kein Umsatz statt.

### Beispiel 12

Es werden 25 g (0,2 mol) Nitrobenzol und 0,5 g AlCl₃ bei 80°C vorgelegt und innerhalb ca. 2 Stunden unter Bedingungen wie in Beispiel 1 bromiert. Man erhält 33 g 3-Bromnitrobenzol. Bei einem Umsatz von 89 %, bezogen auf Nitrobenzol, entspricht das einer Selektivität von 92 % und einer Ausbeute von 82 %.

### Beispiel 13

Es wird wie in Beispiel 1 gearbeitet, jedoch werden 10,008 kg (35,42 mol) 2,4-Dichlor-5-fluorbenzotrichlorid, 194 g Aluminiumchlorid zusammen mit 5,066 kg Brom vorgelegt und bei 20°C beginnend innerhalb von 6 Stunden 1,863 kg Chlor eingeleitet, wobei die Temperatur durch Kühlung auf ca. 30 bis 40°C gehalten wird. Man treibt überschüssiges Halogen mit Stickstoff aus und erhält 12,137 kg eines Rohprodukts mit einer Reinheit von 92 % an 3-Brom-2,4-dichlor-5-fluorbenzotrichlorid, das direkt in die unter Beispiel 2 beschriebene partielle Hydrolyse zum Säurechlorid eingesetzt werden kann.

## Patentansprüche

1. Verfahren zur Bromierung aromatischer Verbindungen, dadurch gekennzeichnet, daß die Bromierung in Anwesenheit eines Aluminiumchlorid-Katalysators sowie Brom und Chlor durchgeführt wird.

2. Verfahren gemäß Anspruch 1, dadurch gekennzeichnet, daß man die zu bromierende Verbindung zusammen mit dem Aluminiumchlorid-Katalysator und dem Brom vorlegt und das Chlor zudosiert.

3. Verfahren nach Anspruch 1, dadurch gekennzeichnet, daß man die zu bromierende Verbindung zusammen mit dem Aluminiumchlorid-Katalysator vorlegt und aus einem zweiten Gefäß vorgewärmtes Brom mit einem Chlorstrom in das Reaktionsgefäß mit der zu bromierenden Verbindung transportiert.

4. Verfahren nach Ansprüchen 1 bis 3, dadurch gekennzeichnet, daß man Brom in einer Menge von 25 bis 200 Mol-% bezogen auf die zu bromierende Verbindung sowie Chlor in einer Menge von ca. 15 bis 190 Mol-% bezogen auf die zu bromierende Verbindung, einsetzt.

5. Verfahren nach Ansprüchen 1 bis 4, dadurch gekennzeichnet, daß man den Aluminiumchlorid-Katalysator in einer Menge von 0,05 bis 10 Mol-% bezogen auf die zu bromierende Verbindung einsetzt.

6. 3-Brom-2,4-dichlor-5-fluorbenzotrichlorid

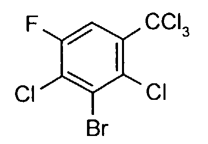

7. Verfahren zur Herstellung der Verbindung nach Anspruch 6, dadurch gekennzeichnet, daß man eine Verbindung der allgemeinen Formel 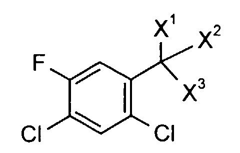 in der
X¹, X² und X³ für drei Chlor stehen,
in Gegenwart von Aluminiumchlorid-Katalysatoren und Chlor bromiert.

8. Verwendung der Verbindung nach Anspruch 6 als Zwischenprodukt bei der Synthese von Chinoloncarbonsäuren.

## Claims

1. Process for the bromination of aromatic compounds, characterized in that the bromination is carried out in the presence of an aluminium chloride catalyst and also bromine and chlorine.

2. Process according to Claim 1, characterized in that the compound to be brominated is introduced as initial charge together with the aluminium chloride catalyst and the bromine, and the chlorine is metered in.

3. Process according to Claim 1, characterized in that the compound to be brominated is introduced as initial charge together with the aluminium chloride catalyst and preheated bromine is transported from a second vessel into the reaction vessel containing the compound to be brominated, using a stream of chlorine.

4. Process according to Claims 1 to 3, characterized in that bromine is employed in a quantity of from 25 to 200 mol%, based on the compound to be brominated, and chlorine is employed in a quantity of from about 15 to 190 mol%, based on the compound to be brominated.

5. Process according to Claims 1 to 4, characterized in that the aluminium chloride catalyst is employed in a quantity of from 0.05 to 10 mol%, based on the compound to be brominated.

6. 3-Bromo-2,4-dichloro-5-fluorobenzotrichloride

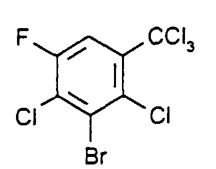

7. Process for the preparation of the compound according to Claim 6, characterized in that a compound of the general formula 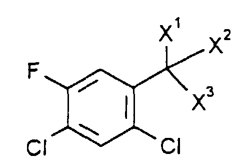 in which
X¹, X² and X³ represent three chlorines,
are brominated in the presence of aluminium chloride catalysts and chlorine.

8. Use of the compound according to Claim 6 as an intermediate in the synthesis of quinolonecarboxylic acids.

## Revendications

1. Procédé pour bromer des composés aromatiques, caractérisé en ce que la bromuration est réalisée en présence d'un catalyseur à base de chlorure d'aluminium, de brome et de chlore.

2. Procédé selon la revendication 1, caractérisé en ce que l'on part du composé à bromer, du catalyseur à base de chlorure d'aluminium et du brome et on ajoute le chlore.

3. Procédé selon la revendication 1, caractérisé en ce que l'on part du composé à bromer et du catalyseur à base de chlorure d'aluminium et on envoie dans le récipient de réaction contenant le composé à bromer, à l'aide d'un courant de chlore, du brome provenant d'un deuxième récipient et chauffé au préalable.

4. Procédé selon les revendications 1 à 3, caractérisé en ce que le brome est mis en oeuvre en quantité de 25 à 200 mol % par rapport au composé à bromer et le chlore en quantité d'environ 15 à 190 mol % par rapport au composé à bromer.

5. Procédé selon les revendications 1 à 4, caractérisé en ce que le catalyseur à base de chlorure d'aluminium est mis en oeuvre en quantité de 0,05 à 10 mol % par rapport au composé à bromer.

6. Le 3-bromo-2,4-dichloro-5-fluorobenzotrichlorure

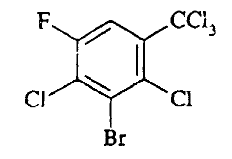

7. Procédé de préparation du composé selon la revendication 6, caractérisé en ce que l'on brome un composé de formule générale 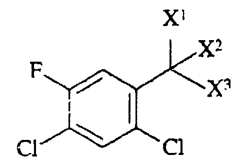 dans laquelle
X¹, X² et X³ représentent chacun un atome de chlore,
en présence de catalyseurs à base de chlorure d'aluminium et de chlore.

8. Utilisation du composé selon la revendication 6 en tant que produit intermédiaire de la synthèse d'acides quinolonecarboxyliques.
